# EUROPEAN PATENT APPLICATION

(11) **EP 2 749 279 A1**
(43) Date of publication of application: **02.07.2014**
(21) Application number: 12824978.6
(22) Date of filing: 24.08.2012
(51) Int. Cl.: A61K 31/167, A61K 9/70, A61K 47/10, A61K 47/32, A61P 29/00, A61P 31/22

(54) **HYDROUS ADHESIVE SKIN PATCH**

(30) Priority: 25.08.2011 US 201161527325 P
(71) Applicant: NIPRO Patch Co., Ltd., Saitama 344-0057 (JP)
(72) Inventor: KAWAMURA, Naohisa, Kasukabe-shi Saitama 344-0057 (JP); RYOO, Je Phil, Monmouth Junction, New Jersey 08852 (US)
(74) Representative: Papula Oy
(86) International application number: PCT/JP2012/071510
(87) International publication number: WO 2013/027840

(57) **Abstract**

The purpose of the present invention is to provide a hydrous adhesive skin patch which contains lidocaine and can be applied in a uniform thickness easily. This hydrous adhesive skin patch comprises a support and an adhesive agent layer arranged on the support, wherein lidocaine or a pharmacologically acceptable salt thereof, a hydrophilic adhesive agent, and diethylene glycol or a diethylene glycol monoalkyl ether are contained in the adhesive agent layer. The diethylene glycol monoalkyl ether is preferably at least one compound selected from the group consisting of diethylene glycol monomethyl ether, diethylene glycol monoethyl ether and diethylene glycol monobutyl ether.

## Description

### TECHNICAL FIELD

The present invention relates to a hydrous adhesive skin patch that contains a drug.

### BACKGROUND ART

Conventionally, percutaneous absorption of a drug using a hydrous adhesive skin patch has been performed for the purpose of the controlled release or local supply of drugs, and the like. For example, it is known that lidocaine can function as a drug for alleviating persistent pain such as postherpetic neuralgia or caused by herpes zoster, as a local anesthetic, and the like. In such use, controlled release of the drug into the body of a patient and delivery of the drug to a local lesion are particularly important. Therefore, percutaneous absorption is more suitable than other administration routes, such as the oral route or an injection, and thus the adhesive skin patch draws attention (see Patent Documents 1 and 2).

[Patent Document 1] Japanese Patent No. 3115615
[Patent Document 2] Pamphlet of PCT International Publication No. WO2001/47559

### DISCLOSURE OF THE INVENTION

### Problems to be Solved by the Invention

An adhesive skin patch is generally manufactured by applying an adhesive agent onto a film having a wide area (release liner) or a support, and then adjusting the thickness through a member limiting the thickness of the adhesive agent, and then cutting the film or support into an adhesive skin patch having a size suitable for a product. However, a conventional adhesive agent containing lidocaine is difficult to apply in a uniform thickness, and the thickness becomes easily uneven. In addition, an apparatus for manufacturing an adhesive skin patch (particularly the limited member) is subjected to an intense load since the elastic force of the adhesive agent is strong, and thus is liable to break down.

The present invention has been elaborated under the actual circumstances described above, and a purpose of the present invention is to provide a hydrous adhesive skin patch that contains lidocaine and can be easily applied in a uniform thickness.

### Means for Solving the Problems

The present inventors found that either diethylene glycol or diethylene glycol monoalkyl can resolve the above-mentioned problems, and completed the present invention. Specifically, the present invention provides the following.
(1) A hydrous adhesive skin patch comprising a support and an adhesive agent layer arranged on the support, wherein
   lidocaine or a pharmacologically acceptable salt thereof, a hydrophilic adhesive agent and diethylene glycol or diethylene glycol monoalkyl ether are contained in the adhesive agent layer.
(2) The hydrous adhesive skin patch described in (1),
   wherein the diethylene glycol monoalkyl ether is at least one selected from the group consisting of diethylene glycol monomethyl ether, diethylene glycol monoethyl ether and diethylene glycol monobutyl ether.
(3) The hydrous adhesive skin patch described in (1) or (2), wherein the diethylene glycol monoalkyl ether comprises diethylene glycol monoethyl ether.
(4) The hydrous adhesive skin patch described in any one of (1) to (3), which contains up to 30 mass% of water with respect to the adhesive agent layer in the adhesive agent layer.
(5) The hydrous adhesive skin patch described in any one of (1) to (4), wherein the hydrophilic adhesive agent comprises polyacrylates.
(6) The hydrous adhesive skin patch described in any one of (1) to (5), wherein the adhesive agent layer contains 40 mass% or more of polyhydric alcohol with respect to the adhesive agent layer.
(7) A method of treating or preventing herpes zoster or postherpetic neuralgia in a patient, the method comprising a step of applying the hydrous adhesive skin patch described in any one of (1) to (6) onto the skin of the patient.

### Effects of the Invention

According to the present invention, diethylene glycol or diethylene glycol monoalkyl ether is blended (contained) in an adhesive agent layer of a hydrous adhesive skin patch, and thus the adhesive agent is easily applied uniformly, and the elastic force of the adhesive agent decreases despite lidocaine being contained in the process of manufacturing the adhesive skin patch, and thus the load applied to the apparatus for manufacturing the adhesive skin patch is suppressed. Accordingly, a hydrous adhesive skin patch containing lidocaine can be provided that can be applied in a uniform thickness easily.

### BRIEF DESCRIPTION OF THE DRAWINGS

Fig. 1 is a graph that illustrates tanδ of an adhesive skin patch pertaining to an example of the present invention.
Fig. 2 is a graph that illustrates the strain to stress of the adhesive skin patch pertaining to an example of the present invention.
Fig. 3 is a graph that illustrates the plasma lidocaine concentration when the adhesive skin patch pertaining to an example of the present invention was used.
Fig. 4 is a graph that illustrates the area under the blood concentration-time curve (AUC) when the adhesive skin patch pertaining to an example of the present invention was used.

### PREFERRED MODE FOR CARRYING OUT THE INVENTION

Hereinafter, embodiments of the present invention are described, but the present invention is not intended to be limited thereto.

The hydrous adhesive skin patch pertaining to the present invention comprises a support, and an adhesive agent layer that contains lidocaine arranged on this support. Details of each element are described below.

### [Adhesive agent layer]

In the hydrous adhesive skin patch of the present invention, lidocaine or a pharmacologically acceptable salt thereof, a hydrophilic adhesive agent and diethylene glycol or diethylene glycol monoalkyl ether are blended in the adhesive agent layer. By this, the adhesive agent is easily applied uniformly, and the elastic force of the adhesive agent decreases despite lidocaine being contained in the process of manufacturing the adhesive skin patch, and thus the load applied to the apparatus for manufacturing the adhesive skin patch is suppressed. Meanwhile, "uniform" in the specification refers to uniformity of the thickness of the adhesive agent layer in the manufacturing process, namely, uniformity of the amount of the adhesive agents among the adhesive agents of respective adhesive skin patches (obtained by cutting in the manufacturing process) manufactured with an identical lot.

Lidocaine is known as a drug for alleviating persistent pain such as postherpetic neuralgia or pain caused by herpes zoster, a local anesthetic or an antiarrhythmic drug, and is a useful drug listed in the Japanese pharmacopoeia. The lidocaine contained in the adhesive agent layer is mainly in a free form, but may be in a form of a pharmacologically acceptable salt. The pharmacologically acceptable salt is not particularly limited, but is preferably a hydrochloric acid salt.

The blending amount (content) of lidocaine and/or a pharmacologically acceptable salt thereof (hereinafter, also referred to as lidocaines) is not particularly limited. However, if the blending amount is too high, lidocaines are severely precipitated, and difficult to keep in the formulation. If the blending amount is too low, sufficient actions are hardly obtained. Accordingly, the blending amount of lidocaines is 0.1 mass% to 50 mass%, preferably 1.0 mass% to 30 mass%, more preferably 3 mass% to 10 mass% and most preferably 5 mass% with respect to the total mass of the adhesive agent layer. The present invention is advantageous in the points that the adhesive agent is easily applied uniformly, and the elastic force of the adhesive agent can be decreased despite lidocaine being contained in the above-mentioned amount range.

The diethylene glycol or diethylene glycol monoalkyl ether may be used in one kind or in combination thereof, but at least diethylene glycol monoalkyl ether is preferably contained with a view to maximizing the effects of the present invention.

The diethylene glycol monoalkyl ether is not particularly limited, but may be, for example, at least one selected from the group consisting of diethylene glycol monomethyl ether, diethylene glycol monoethyl ether and diethylene glycol monobutyl ether. Among them, diethylene glycol monoethyl ether is preferably contained with a view to maximizing the effects of the present invention. In addition, when the hardness of the adhesive agent is decreased at the time of the application, the adhesive force of the adhesive agent layer of the adhesive skin patch after manufacture generally tends to decrease. However, diethylene glycol monoethyl ether is advantageous in that it can resolve these problems compatibly.

If the blending amount of diethylene glycol and diethylene glycol monoalkyl ether is too small, the effects of the present invention are hardly sufficiently obtained. On the other hand, if the blending amount of diethylene glycol and diethylene glycol monoalkyl ether is too great, the liquid components (diethylene glycol monoalkyl ether and the like) are easily exuded (bleeding) from the adhesive agent layer. Therefore, the sum of the blending amounts of diethylene glycol and diethylene glycol monoalkyl ether is preferably 0.5 mass% or more, more preferably 1 mass% or more and 10 mass% or less, and more preferably 5 mass% or less, and even more preferably 3 mass% or less with respect to the adhesive agent layer.

The blending amount of water is not particularly limited as long as it is acceptable in the manufacture of the formulation. If the blending amount of water in the adhesive agent is low, the adhesive agent tends to be hard and difficult to apply. However, the present invention is advantageous in that such defects can be suppressed. Therefore, the blending amount of water in the adhesive agent layer is a range of 40 mass% or less, preferably 30 mass% or less, and more suitably 28 mass% or less with respect to the adhesive agent layer. However, the blending amount of water may be 40 mass% or more with respect to the adhesive agent layer. Meanwhile, the lower limit of the water content may be suitably set up considering that if the lower limit of the water content is too low, the blending amount of other components (for example, glycerol and the like) increases and the manufacturing cost increases, and sufficient amounts of other components (for example, polyacrylate and/or a salt thereof) are not dissolved, and the like. Generally, the lower limit of the water content is preferably 10 mass%, and more preferably 15 mass% with respect to the total mass of the adhesive agent layer.

The hydrophilic adhesive agent is not particularly limited if it can be used in the hydrous adhesive skin patch, but is preferably a polyacrylate. Examples of the polyacrylates include polyacrylate, sodium polyacrylate, and partially neutralized polyacrylate (for example "NP-800 (trademark)" and "NP-700 (trademark)" (manufactured by Showa Denko K.K.)), and these may be included alone in one kind or in combination of two or more kinds.

The blending amount of polyhydric alcohol may be also suitably selected from the above-mentioned ranges, but is preferably 40 mass% or more, and more preferably 50 mass% or more with respect to the total mass of the adhesive agent layer from the viewpoints that an increase of the adhesive force can be suppressed and excellent re-bonding force can be further improved. Meanwhile, the upper limit of the blending amount of polyhydric alcohol may be suitably set up in consideration of the blending amount of water, manufacturing cost and easiness of occurrence of bleeding, and the like.

Examples of the polyhydric alcohol include glycerol, sorbitol, ethylene glycol, propylene glycol, polyethylene glycol, polypropylene glycol, 1,3-butylene glycol, 1,3-propanediol, 1,3-butanediol, 1,4-butanediol, 1,2,6-hexanetriol, maltitol and xylitol, and these may be contained alone in one kind or in a combination of two or more kinds. Among them, the polyhydric alcohol is preferably glycerol, sorbitol or propylene glycol. Examples of the glycerol include conc. glycerol (98.0 to 101.0% of the glycerol content) and other glycerols (84 to 87% of the glycerol content), but are not particularly limited. However, the glycerol is more preferably conc. glycerol (98.0 to 101.0% of the glycerol content).

The adhesive agent layer may contain optional components as necessary, such as inorganic powders, a surfactant, a crosslinking agent, a crosslinking-controlling agent, an adhesion enhancer, a solubilizing agent for lidocaines, a pH regulating agent, a refreshing agent, an aqueous polymer compound, inorganic powders, an antioxidant, an antiseptic, a pigment and a moisturizing agent in addition to the above-mentioned components.

Examples of the inorganic powders include kaolin, zinc oxide, titanium oxide, silicic acid anhydride, light anhydrous silicic acid and the like, and these may be included alone in one kind or in combination of two or more kinds. Among them, at least one of titanium oxide and silicic acid anhydride is preferably included. The inorganic powders suppress excessive increase of the adhesive force. Accordingly, the blending amount of inorganic powders is preferably such an amount that the sum of the blending amount of inorganic powders and the blending amount of the polyhydric alcohol is 50 mass% or more with respect to the total mass of the adhesive agent layer.

The surfactant is not particularly limited, but

Examples thereof include sorbitan fatty acid esters such as sesquioleate acid sorbitan, monolauric acid sorbitan, monopalmitic acid sorbitan and monostearate acid sorbitan; glycerol fatty acid esters such as glyceryl monostearate; polyglycerol fatty acid esters such as monolauric acid hexaglycerol and decaoleic acid decaglycerol; polyethylene glycol fatty acid esters such as distearate acid polyethylene glycol and monostearate acid polyethylene glycol; polyoxyethylene sorbitan fatty acid esters such as triolein acid polyoxyethylene sorbitan and mono-oleic acid polyoxyethylene sorbitan; polyoxyethylene sorbitol fatty acid esters such as tetraolein acid polyoxyethylene sorbitol; polyoxyethylene glycerol fatty acid esters such as mono-oleic acid polyoxyethylene glyceryl; polyoxyethylene alkyl ethers such as polyoxyethylene lauryl ether and polyoxyethylene cetyl ether; polyoxyethylene polyoxypropylene alkyl ethers such as polyoxyethylene polyoxypropylene cetyl ether and polyoxyethylene polyoxypropylene decyl tetradecyl ether; polyoxyethylene fatty acid amides such as polyoxyethylene stearic acid amide; and surfactants such as polyoxyethylene castor oil and polyoxyethylene hydrogenated castor oil, and these may be included alone in one kind or in combination of two or more kinds. The surfactant is not particularly limited, but is preferably polyoxyethylene sorbitan fatty acid ester, and suitably triolein acid polyoxyethylene sorbitan or mono-oleic acid polyoxyethylene sorbitan (Polysorbate 80).

The pH of the adhesive agent layer is preferably adjusted to 4.5 to 9, and more preferably 6 to 7.4. If the pH of the adhesive agent layer is less than 4.5, migration of a drug, particularly lidocaine to the skin becomes worse. Accordingly, the pH of the adhesive agent layer is desirably 4.5 or more, but with such a weak acidic pH level, a crosslinking agent different to those generally used, specifically dihydroxyaluminum aminoacetate (another name; aluminum glycinate), is preferably used. The aluminum of dihydroxyaluminum aminoacetate is well eluted in the vicinity of weak acidic to neutral pH, and can be well crosslinked with the polyacrylate (salt) described below and the like.

Meanwhile, examples of the crosslinking agent are not limited to dihydroxyaluminum aminoacetate, but include polyvalent metal salts, preferably aluminum compounds, among them. Examples of the aluminum compounds include hydroxides such as dihydroxyaluminum aminoacetate described above, aluminum hydroxide and aluminum hydroxide gel; or salts of inorganic acid or organic acid such as aluminum chloride, aluminum sulfate, aluminum acetate and aluminum stearate; double salts such as aluminum Alums; aluminate salts such as sodium aluminate; inorganic aluminum complex salts; and organic aluminum chelate compounds; and the like. These aluminum compounds may be aqueous, or may be poorly soluble.

Meanwhile, the pH may be set up using a pH adjusting agent, and examples of such pH adjusting agent may include tartaric acid, phosphoric acid, malic acid, citric acid, hydrochloric acid, sodium hydroxide, triethanol amine, diethanolamine, diisopropanolamine and the like, and these may be included alone in one kind or in a combination of two or more kinds. The pH adjusting agent is preferably tartaric acid or phosphoric acid.

Examples of the adhesion enhancer include n-butyl methacrylate·acrylate copolymers, methyl acrylate·2-ethylhexyl acrylate copolymers, polybutene, ester gum, terpene resins, aliphatic saturated hydrocarbon resin and the like. The blending amount thereof may be 1 mass% or more and 30 mass% or less, and preferably 5 mass% or more and 20 mass% or less with respect to the total mass of the adhesive agent layer.

Examples of the crosslinking-controlling agent (chelating agent) include sodium edetate (ethylene diamine tetraacetate disodium), citric acid and the like, and these may be included alone in one kind or in a combination of two or more kinds. The crosslinking-controlling agent is preferably sodium edetate.

Examples of the solubilizing agent for lidocaines include crotamiton, N-methyl-2-pyrrolidone, peppermint oil, 1,3-butylene glycol, diethylene glycol monoethyl ether and the like, and these may be included alone in one kind or in combination of two or more kinds.

Examples of the refreshing agent include camphor and thymol, and in addition, menthol derivatives such as l-menthol, dl-menthol, 2-methyl-3-(l-menthyloxy)propane-1,2-diol, 3-1-menthoxypropane-1,2-diol and 5-methyl-2-(l-methylethyl)-cyclohexyl-2-hydroxypropionate, and the like, and these may be included alone in one kind or in a combination of two or more kinds.

Examples of the aqueous polymer compound (thickener for the aqueous polymer compound) include gelatin, agar, polyvinyl alcohol, polyvinyl pyrrolidone, propylene carbonate, carboxymethyl cellulose, sodium carboxymethyl cellulose, hydroxypropyl cellulose, hydroxypropyl methyl cellulose, hydroxyethyl cellulose, methyl cellulose, sodium alginate, maleic acid anhydride copolymers, carrageenan and the like, and these may be included alone in one kind or in a combination of two or more kinds.

Examples of the antioxidant include tocopherol acetate, ascorbic acid and/or derivatives thereof, sodium sulfite, sodium hydrogen sulfite, sodium pyrosulfite, sodium nitrite, dibutylhydroxytoluene and the like, and these may be included alone in one kind or in a combination of two or more kinds.

Examples of the antiseptic (preservative) include methyl parahydroxybenzoate, ethyl parahydroxybenzoate, propyl parahydroxybenzoate, butyl parahydroxybenzoate, thymol and the like, and these may be included alone in one kind or in a combination of two or more kinds.

Examples of the pigment are not particularly limited for the kind, but include pigments described in legal pigment handbooks, and these may be used alone in one kind or in a combination of two or more kinds.

Examples of the moisturizing agent are not particularly limited as long as they are those generally used, but include xylitol, sorbitol, maltitol, pyrrolidone carboxylic acid, sodium pyrrolidone carboxylic acid, sodium lactate, hyaluronic acid, sodium hyaluronate, urea and the like. In addition, the above-mentioned polyhydric alcohol also functions as a moisturizing agent.

### [Support]

The support may consist of fabric such as woven fabric, nonwoven fabric and knitted fabric, a resin film, paper and a laminate thereof, which are conventionally known and used for an adhesive skin patch. A material for the support may be one kind or two or more kinds selected from the group consisting of polypropylene, polyethylene, polybutylene, polyethylene terephthalate, rayon, cotton and polyurethane, and is not particularly limited, but is preferably polyethylene terephthalate. A support made of nonwoven fabric consisting of polyethylene terephthalate is preferably used in terms of the cost. In addition, a support colored by printing or kneading a paint with a white color, flesh color and the like, or a support with letters and the like may be used when the resin film is used, or a support treated with polyurethane treatment, matting treatment and the like may be used in order to improve the anchoring property of the adhesive agent.

### [Release liner]

The adhesive skin patch pertaining to the present invention may further comprise a release liner that coats the adhesive agent layer. Such release liner is preferably a film of a resin such as polyethylene terephthalate and polypropylene, and those with release treatment of silicon, and the like, or those with an embossing process applied may be used as such release liner. In addition, those printed or kneaded with a white paint or the like may be also used as a release liner.

The adhesive skin patch described above may be used for any use, but particularly, preferably used in the treatment or prevention of persistent pain such as postherpetic neuralgia or pain caused by herpes zoster.

### [Preparation method]

The adhesive skin patch of the present invention can be prepared by a conventional method, and can be prepared by suitably blending the essential components and the optional components as necessary and kneading the blend uniformly with a known method, and spreading it onto a release liner such that the mass of the adhesive agent per unit area of the adhesive skin patch is 0.03 to 0.15 g/cm², and then also laminating a support onto the surface of the adhesive agent layer, and then cutting the laminate into a rectangular shape into 100 mm x 140 mm. In addition, the adhesive skin patch of the present invention can be also prepared by spreading the adhesive agent previously on the support, and then laminating a release liner thereon.

The present invention is a method for treating or preventing herpes zoster or postherpetic neuralgia in a patient, and includes a step of applying the hydrous adhesive skin patch described above onto the skin of the patient. Since the adhesive agent is uniformly applied in the hydrous adhesive skin patch of the present invention, the use of such an adhesive skin patch permits the obtaining of homogeneous effects as expected in the treatment or prevention of herpes zoster or postherpetic neuralgia.

Hereinafter, the present invention will be specifically described showing Examples and Comparative Examples. However, the present invention is not limited to Examples.

### EXAMPLES

### Example 1

The respective components shown in Table 1 were stirred and mixed for a certain time, and then applied onto a release liner (long shape having a width corresponding to 6 rows of the adhesive skin patch) such that the mass of the adhesive agent per 1 piece of the adhesive skin patch (140 mm x 100 mm) was about 14 g, and passed under a tabular member of which the distance from the film was a prescribed distance, whereby to unify the thickness of the adhesive agent layer. Then, on the surface of the adhesive agent layer, nonwoven fabric made of polyethylene terephthalate was laminated, and then cut into a size of 100 mm x 140 mm whereby to prepare adhesive skin patches.

More specifically, in accordance with each component and quantity in Table 1, conc. glycerol, tartaric acid, phosphoric acid, carboxymethyl cellulose sodium, partially neutralized polyacrylate, gelatin, dihydroxyaluminum aminoacetate, sodium edetate and a suitable amount of purified water were uniformly mixed to prepare a hydrous gel. Then, lidocaine, methyl parahydroxybenzoate and propyl parahydroxybenzoate were dissolved in diethylene glycol monoethyl ether and Polysorbate 80, and then the solution was uniformly dispersed in the previously prepared hydrous gel to obtain an adhesive agent composition. This adhesive agent composition was spread onto a release liner (release film), and the surface of the adhesive agent layer was coated with nonwoven fabric made of polyethylene terephthalate whereby to prepare adhesive skin patches. Meanwhile, the amounts shown in Table 1 are based on mass%.

### Example 2 and Comparative Example 1

For Example 2 and Comparative Example 1, the adhesive skin patches were prepared in a similar method to Example 1 mentioned above except the blend shown in Table 1. Meanwhile, the amounts shown in Table 1 are based on mass%.

**[Table 1]**

| | | Example 1 | Example 2 | Comparative Example 1 |
|---|---|---|---|---|
| Active ingredient | Lidocaine | 5 | 5 | 5 |
| Solubilizing agent | Diethylene glycol monoethyl ether (^{\|}Transcutol┘ (Trademark)) | 2 | 2 | - |
| Adhesive | Partially neutralized polyacrylate | 4 | 4 | 4 |
| Viscosity enhancer | Carboxymethyl cellulose sodium | 2 | 2 | 2 |
| Viscosity enhancer | Gelatin | 2 | 2 | 2 |
| Cross-linking agent | Dihydroxyaluminun aminoacetate | 0.5 | 0.5 | 0.5 |
| Humectant | Conc. glycerol | 53.85 | 53.80 | 55.85 |
| pH adjustment | Tartaric acid | 0.83 | 0.83 | 0.83 |
| pH adjustment | Phosphoric acid | 0.02 | 0.02 | 0.02 |
| Emulsifier | Polysorbate 80 (Mono-oleic acid polyoxyethylene sorbitan) | 0.3 | 0.3 | 0.3 |
| Chelating agent | Sodium edetate | 0.15 | 0.15 | 0.15 |
| Preservative | Methyl parahydroxybenzoate | 0.1 | 0.1 | 0.1 |
| Preservative | Propyl parahydroxybenzoate | 0.05 | 0.05 | 0.05 |
| Diluent | Titanium oxide | 0.2 | 0.2 | 0.2 |
| Diluent | Light anhydrous silicic acid | 1 | 1 | 1 |
| Diluent | Dry sulfite sodium | - | 0.05 | - |
| Diluent | Purified water | 28 | 28 | 28 |
| | Sum | 100 | | |

### Examples 3 to 5 and Comparative Example 2

For Examples 3 to 5 and Comparative Example 2, the adhesive skin patches were prepared in a similar method to Example 1 mentioned above except the blend shown in Table 2. Meanwhile, the amounts shown in Table 2 are based on mass%.

**[Table 2]**

| | | Comparative Example 2 | Example 3 | Example 4 | Example 5 |
|---|---|---|---|---|---|
| Active ingredient | Lidocaine | 5 | 5 | 5 | 5 |
| Solubilizing agent | Diethylene glycol monoethyl ether (^{\|}Transcutol┘ (Trademark)) | - | 3 | 3 | 3 |
| Adhesive | Partially neutralized polyacrylate | 4 | 4 | 4 | 4 |
| Viscosity enhancer | Carboxymethyl cellulose sodium | 4 | 4 | 4 | 4 |
| Viscosity enhancer | Gelatin | 1 | 1 | 1 | 1 |
| Viscosity enhancer | Poll-vinyl alcohol | 1 | 1 | 1 | 1 |
| Cross-linking agent | Dihydroxyaluminum aminoacetate | 0.5 | 0.5 | 0.5 | 0.5 |
| Humectant | Propylene glycol | 5 | 5 | 5 | 5 |
| Humectant | Sorbitol | 15 | 15 | 15 | 15 |
| Humectant | Conc. glycerol | 20 | 31 | 20 | 20 |
| pH adjustment | Tartaric acid | 1.18 | 0.83 | 0.83 | 1.3 |
| pH adjustment | Phosphoric acid | - | 0.02 | 0.02 | 0.02 |
| Emulsifier | Polysorbate 80 (Mono-oleic acid polyoxyethylene sorbitan) | - | 0.3 | 0.3 | - |
| Chelating agent | Sodium edetate | 0.8 | 0.2 | 0.4 | 0.6 |
| Presevative | Methyl parahydroxybenzoate | 0.1 | 0.1 | 0.1 | 0.1 |
| Preservative | Propyl parahydroxybenzoate | 0.05 | 0.05 | 0.05 | 0.05 |
| Humectant | Urea | 1 | 1 | 1 | |
| Diluent | Kaolin | 1 | | | |
| Diluent | Purified water | 40.37 | 28 | 38.8 | 39.43 |
| | Sum | 100 | | | |

### (Test Example 1)

The mass of the adhesive agent in each adhesive skin patch of Examples and Comparative Examples was measured. The results are shown in Tables 3 (Example 1) and 4 (Comparative Example 1), respectively. As shown in Tables 3 and 4, the adhesive skin patch of Example 1 had a uniform amount of the adhesive agent, and had low unevenness in comparison to the adhesive skin patch of Comparative Example 1. Meanwhile, the adhesive skin patches of Examples 2 to 5 also had a uniform amount of the adhesive agent, and had low unevenness in the equivalent degree to Example 1, although not shown in the Table. On the other hand, the adhesive skin patch of Comparative Example 2 showed a similar value to Comparative Example 1, and great unevenness of the adhesion amount.

**[Table 3]**

| Row applied | | Row 1 | Row 2 | Row 3 | Row 4 | Row 5 | Row 6 |
|---|---|---|---|---|---|---|---|
| Piece 1 | | 14.08 | 13.35 | 13.22 | 13.59 | 13.76 | 13.20 |
| Piece 2 | | 14.12 | 13.51 | 13.22 | 13.80 | 13.33 | 13.30 |
| Piece 3 | | 14.04 | 13.83 | 13.46 | 14.02 | 13.72 | 13.29 |
| Piece 4 | | 14.17 | 13.61 | 13.45 | 13.98 | 13.91 | 13.33 |
| Piece 5 | | 14.23 | 13.41 | 13.41 | 13.27 | 13.70 | 13.86 |
| Piece 6 | | 14.18 | 14.12 | 14.29 | 14.18 | 13.36 | 13.98 |
| Piece 7 | | 14.19 | 13.59 | 14.24 | 14.15 | 13.17 | 13.91 |
| Piece 8 | | 14.28 | 13.59 | 13.87 | 14.21 | 13.88 | 13.62 |
| Piece 9 | | 14.19 | 13.71 | 13.46 | 13.88 | 13.25 | 13.75 |
| Piece 10 | | 13.87 | 13.68 | 13.60 | 13.98 | 13.30 | 13.48 |
| Piece 11 | | 13.89 | 13.44 | 14.36 | 14.09 | 13.17 | 13.90 |
| Piece 12 | | 13.92 | 14.09 | 13.24 | 13.90 | 13.00 | 13.83 |
| Piece 13 | | 13.96 | 14.60 | 13.32 | 13.64 | 13.15 | 13.58 |
| Piece 14 | | 14.17 | 13.61 | 13.18 | 13.36 | 13.88 | 13.61 |
| Piece 15 | | 14.15 | 13.60 | 13.31 | 13.21 | 13.99 | 13.65 |
| Piece 16 | | 13.98 | 13.23 | 13.24 | 13.18 | 13.79 | 13.71 |
| Piece 17 | | 13.84 | 13.66 | 13.19 | 13.57 | 14.06 | 13.67 |
| Piece 18 | | 14.77 | 14.04 | 13.36 | 13.11 | 13.27 | 13.85 |
| Piece 19 | | 14.42 | 14.10 | 13.25 | 13.35 | 14.25 | 13.78 |
| Piece 20 | | 14.61 | 14.54 | 13.83 | 13.32 | 14.23 | 13.22 |
| Mass of paste (g) | maximum value | 14.77 | | | | | |
| | minimum value | 13.00 | | | | | |
| | average value | 13.73 | | | | | |
| | RSD | 2.8 | | | | | |
| Ratio of mass of paste (%) | maximum value | 105.5 | | | | | |
| | minimum value | 92.9 | | | | | |
| | difference (maximum-minimum) | 12.6 | | | | | |

**[Table 4]**

| Row applied | | Row 1 | Row 2 | Row 3 | Row 4 | Row 5 | Row 6 |
|---|---|---|---|---|---|---|---|
| Piece 1 | | 12.69 | 13.43 | 12.36 | 13.46 | 12.31 | 13.38 |
| Piece 2 | | 12.60 | 12.96 | 12.17 | 13.32 | 14.31 | 12.82 |
| Piece 3 | | 12.97 | 13.28 | 12.44 | 12.98 | 13.44 | 13.15 |
| Piece 4 | | 13.49 | 12.95 | 12.92 | 12.86 | 12.60 | 13.58 |
| Piece 5 | | 13.98 | 13.60 | 13.78 | 13.28 | 14.35 | 14.17 |
| Piece 6 | | 13.81 | 14.01 | 14.00 | 12.90 | 13.59 | 14.25 |
| Piece 7 | | 13.30 | 13.81 | 13.52 | 12.14 | 12.06 | 14.43 |
| Piece 8 | | 13.60 | 13.08 | 13.14 | 15.16 | 12.30 | 15.07 |
| Piece 9 | | 13.56 | 12.12 | 13.64 | 15.50 | 12.62 | 16.05 |
| Piece 10 | | 12.95 | 12.86 | 13.55 | 14.81 | 12.99 | 15.05 |
| Piece 11 | | 12.69 | 11.81 | 14.02 | 13.42 | 13.51 | 15.28 |
| Piece 12 | | 12.67 | 13.27 | 13.47 | 13.05 | 13.22 | 14.95 |
| Piece 13 | | 12.76 | 13.39 | 14.20 | 12.87 | 13.67 | 14.50 |
| Piece 14 | | 12.91 | 13.48 | 13.93 | 13.53 | 14.66 | 13.79 |
| Piece 15 | | 12.86 | 13.11 | 13.07 | 13.07 | 15.06 | 14.02 |
| Piece 16 | | 12.75 | 12.13 | 12.92 | 13.02 | 15.07 | 13.63 |
| Piece 17 | | 12.51 | 12.80 | 12.92 | 12.75 | 14.92 | 13.61 |
| Piece 18 | | 12.59 | 13.04 | 13.05 | 13.28 | 14.60 | 14.15 |
| Piece 19 | | 12.66 | 13.00 | 13.13 | 13.63 | 14.60 | 14.21 |
| Piece 20 | | 12.67 | 12.63 | 13.74 | 13.08 | 15.19 | 12.27 |
| Mass of paste (g) | maximum value | 13.98 | 14.01 | 14.20 | 15.50 | 15.19 | 15.28 |
| | minimum value | 12.51 | 11.81 | 12.17 | 12.14 | 12.06 | 12.27 |
| | average value | 13.00 | 13.04 | 13.30 | 13.41 | 13.75 | 14.07 |
| | RSD | 3.47 | 4.27 | 4.36 | 6.19 | 7.60 | 5.74 |
| | maximum value | 15.50 | | | | | |
| | minimum value | 11.81 | | | | | |
| | average value | 13.43 | | | | | |
| | RSD | 6.09 | | | | | |
| Ratio of mass of paste | maximum value | 110.7 | | | | | |
| | minimum value | 84.4 | | | | | |
| | difference (maximum-minimum) | 26.4 | | | | | |

### (Test Example 2)

The dynamic shear elastic modulus (G') and the like of the adhesive agent in the adhesive skin patches of Example 1 and Comparative Examples were measured using a Rheometrics Dynamic Analyzer "Dynamic Analyzer RDAIII (trademark)" (manufactured by Rheometric Scientific).

### "Dynamic viscoelasticity (temperature dependence)"

0.65 to 0.75 g of the adhesive agent was weighed, and put on the center of a parallel plate (25 mm diameter), and sandwiched with a cone plate (25 mm diameter, 0.1 rad angle), and the clearances of both of the plates were set up to 0.05 mm, and tanδ when heated to 30°C to 45°C at the conditions of 1% distortion, 1 Hz frequency and 5°C per minute was measured. Measurement conditions
• Frequency: 1 Hz
• Temperature: 30°C to 45°C (at first set up to 30°C, and heated to 45°C at a rate of 5 °C /minute.)
• Measurement and plotting: Measured and plotted per 12 seconds.
• Plate: parallel plate (25 mm diameter) and cone plate (25 mm diameter, 0.1 rad angle)
• Clearance (Gap): 0.05 mm
• Strain amount: 1%
• PC software (Orchestrator Ver. 6.5.6; manufactured by Rheometric Scientific)

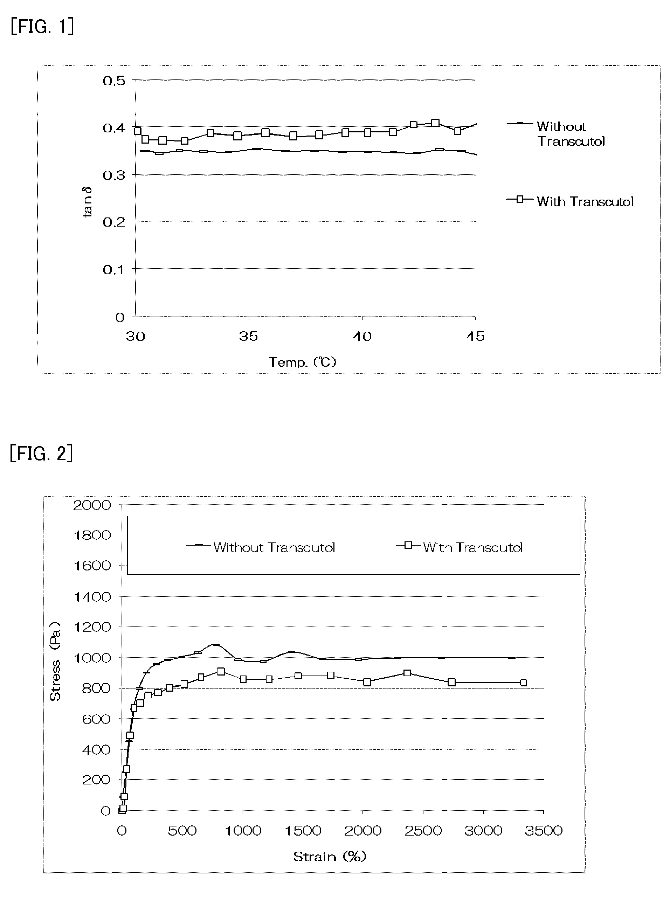

As illustrated in Fig. 1, it was found that the adhesive agent of Example 1 ("With Transcutol") showed high tanδ at any temperature from 30 to 50°C in comparison to the adhesive agent of Comparative Example 1 ("Without Transcutol"), and was soft. It is presumed that 30 to 50 °C is a normal temperature in a step of applying the adhesive agent, and high tanδ (softness) at the temperature contributes to the uniformity of the application and load relief for the manufacturing facility. Meanwhile, the adhesive skin patches of Examples 2 to 5 also showed high tanδ similarly to Example 1, although not illustrated in Fig. 1. On the other hand, the adhesive skin patch of Comparative Example 2 showed low tanδ similarly to Comparative Example 1.

### (Test Example 3)

The dynamic shear elastic modulus (G') and the like of the adhesive agent in the adhesive skin patches of Examples and Comparative Examples were measured using a Rheometrics Dynamic Analyzer similarly to Test Example 2 at the conditions described below, and a stress-strain curve was obtained. The results are illustrated in Fig. 2.

### "Dynamic viscoelasticity (stress-distortion curve)"

0.65 to 0.75 g of the adhesive agent was weighed, and put on the center of a parallel plate (25 mm diameter), and sandwiched with a cone plate (25 mm diameter, 0.1 rad angle), and the clearances of both of the plates were set up to 0.05 mm, and the stress-distortion curve was drawn under the condition of a temperature of 40°C.

### Measurement conditions

• Frequency: 1 Hz
• Temperature: 40°C
• Measurement and plotting: measured and plotted every 12 seconds
• Plate: Parallel plate (25 mm diameter) and cone plate (25 mm diameter, 0.1 rad angle)
• Clearance (Gap): 0.05 mm
• Strain amount: 1%
• PC software (Orchestrator Ver. 6.5.6; manufactured by Rheometric Scientific)

As illustrated in Fig. 2, it was found that the adhesive agent of Example 1 ("With Transcutol") had low stress to strain over a wide range in comparison to the adhesive agent of Comparative Examples ("Without Transcutol"). It is presumed that this mechanical property contributes to the uniformity of the application and load relief for the manufacturing facility. Meanwhile, the adhesive skin patches of Examples 2 to 5 also showed low strain similarly to Example 1, although not illustrated in Fig. 2. On the other hand, the adhesive skin patch of Comparative Example 2 showed high strain similarly to Comparative Example 1.

### (Test Example 4)

The adhesive skin patch of Example was cut to 2.0 x 3.0 cm, and pasted onto the human forearm. The adhesion property and degree of pain at the time when the adhesive skin patch was released after 8 hours from the human forearm, and the re-adhesion property when the adhesive skin patch was pasted again onto the skin after being released from the skin after 8 hours, were sensory-evaluated by 8 monitoring persons. The adhesive skin patches of Examples were excellent for any item.

### (Test Example 5) In vivo percutaneous absorption test in a pig

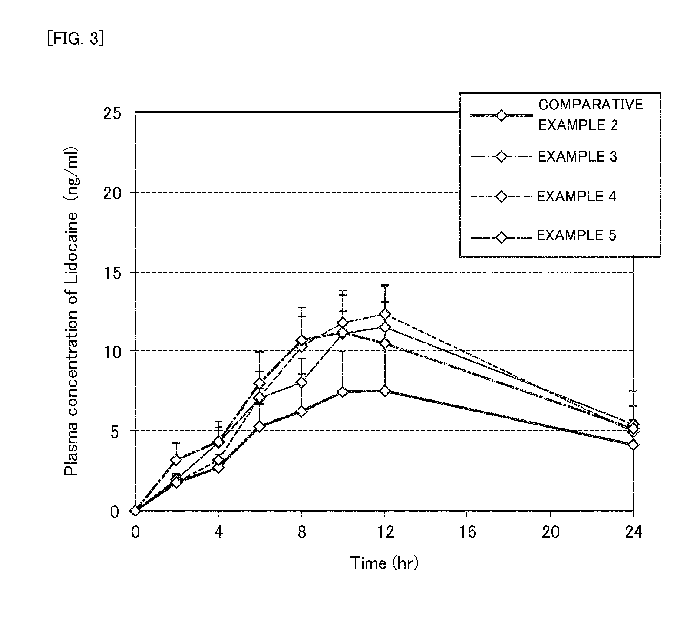

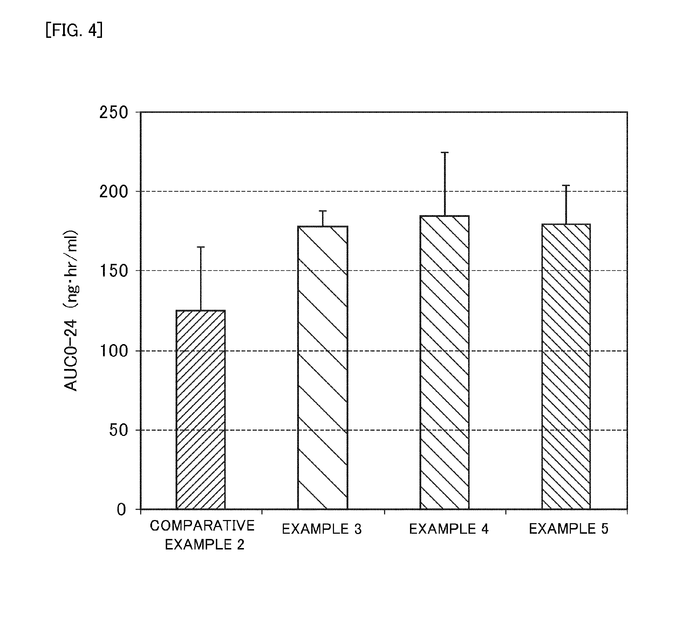

A pig (lineage: Landrace & Large Yorkshire, Nosan Corporation, female, 4 to 5 months old, about 20 kg body weight) had the hair on its back shaved back, and each of 2 pieces of adhesive skin patches for the test cut to 100 mm x 140 mm (Examples 3 to 5 and Comparative Example 2) was pasted for 12 hours. Then, the blood was collected at regular intervals, and the plasma lidocaine concentration was measured. In addition, the area under the blood concentration-time curve (AUC) was calculated from plasma lidocaine concentration for 24 hours. The results are illustrated in Figs. 3 and 4.

As illustrated in Figs. 3 and 4, it was found out that the adhesive skin patches of Examples 3 to 5 showed better plasma lidocaine concentration and AUC in comparison to the adhesive skin patch of Comparative Example 2.

## Claims

1. A hydrous adhesive skin patch comprising a support and an adhesive agent layer arranged on the support,
wherein lidocaine or a pharmacologically acceptable salt thereof, a hydrophilic adhesive agent and diethylene glycol or diethylene glycol monoalkyl ether are contained in the adhesive agent layer.

2. The hydrous adhesive skin patch according to claim 1, wherein the diethylene glycol monoalkyl ether is at least one selected from the group consisting of diethylene glycol monomethyl ether, diethylene glycol monoethyl ether and diethylene glycol monobutyl ether.

3. The hydrous adhesive skin patch according to claim 1 or 2, wherein the diethylene glycol monoalkyl ether comprises diethylene glycol monoethyl ether.

4. The hydrous adhesive skin patch according to any one of claims 1 to 3, which contains 30 mass% or less of water with respect to the adhesive agent layer in the adhesive agent layer.

5. The hydrous adhesive skin patch according to any one of claims 1 to 4, wherein the hydrophilic adhesive agent comprises polyacrylates.

6. The hydrous adhesive skin patch according to any one of claims 1 to 5, which contains 40 mass% or more of polyhydric alcohol with respect to the adhesive agent layer in the adhesive agent layer.

7. A method of treating or preventing herpes zoster or postherpetic neuralgia in a patient, the method comprising a step of applying the hydrous adhesive skin patch according to any one of claims 1 to 6 onto the skin of the patient.
